Europäisches Patentamt

European Patent Office

Office européen des brevets

(19)

(11) EP 0 765 663 A2

(12) **EUROPÄISCHE PATENTANMELDUNG**

(43) Veröffentlichungstag:
02.04.1997 Patentblatt 1997/14

(21) Anmeldenummer: 96114619.8

(22) Anmeldetag: 12.09.1996

(51) Int. Cl.⁶: **A61K 31/565**, A61K 7/48
// (A61K31/565, 31:565)

(84) Benannte Vertragsstaaten:
AT BE CH DE DK ES FI FR GB GR IE IT LI LU MC NL PT SE
Benannte Erstreckungsstaaten:
LT LV SI

(30) Priorität: 15.09.1995 DE 19534209

(71) Anmelder: JENAPHARM GmbH
D-07745 Jena (DE)

(72) Erfinder:
- Golbs, Siegfried, Dr.
  04207 Leipzig (DE)
- Oettel, Michael, Prof. Dr.
  07743 Jena (DE)
- Dittgen, Michael, Prof. Dr.
  99510 Apolda (DE)
- Gräser, Thomas, Dr.
  99095 Erfurt (DE)
- Luderschmidt, Christoph, Prof. Dr.
  81245 München (DE)

(54) **Verwendung von Dienogest zur Herstellung eines Arzneimittels zur Behandlung der Haut mittels topischer Anwendung**

(57) Gegenstand der Erfindung ist die Verwendung des antiandrogen wirkenden Gestagens Dienogest oder einer Kombination von Dienogest und einem Estrogen, gegebenfalls in einer pharmazeutischen Zusammensetzung zur Behandlung der Haut, wobei systemische Nebenwirkungen durch Verhinderung der Permeation des Wirkstoffes durch die Haut vermieden werden.

Die vorliegenden pharmazeutischen Zubereitungen fördern das Eindringen des Wirkstoffes oder der Wirkstoffkombination , schränken jedoch seinen/ihren Durchtritt durch die Haut und die Resorption ein.

Dienogesthaltige pharmazeutische Zusammensetzungen zur transdermalen Applikation zur Behandlung der Haut sind, besonders bei Krankheitsbildern wie Acne vulgaris, Seborrhoe, androgenetisch bedingter Alopezie sowie Androgenisierungssymptomen der Frau und vasomotorischem Kopfschmerz einsetzbar.

**Beschreibung**

Die Erfindung betrifft die Verwendung von Dienogest ausschließlich oder in Kombination mit einem synthetischen oder natürlichen Estrogen oder Phytestrogen zur Herstellung eines Arzneimittels zur Behandlung der Haut , besonders bei Acne vulgaris, Seborrhoe und androgenetisch bedingter Alopezie sowie Androgenisierungssymptomen der Frau, mittels topischer Anwendung, wobei systemische Nebenwirkungen durch Verhinderung der Permeation des Wirkstoffes durch die Haut vermieden werden.

In Mitteleuropa wird die Zahl an hormonal bedingten Hauterkrankungen wie Akne, Seborrhoe, Hirsutismus, auf 35 bzw. 50 % geschätzt. Das Krankheitsbild zeigt sich hauptsächlich bei jungen Männern und Frauen im fertilen Alter, die dadurch einem großen Leidensdruck ausgesetzt sind.

Die Talgdrüsenaktivität wird durch Hormone wesentlich beeinflußt. Testosteron und Dihydrotestosteron sind für die Sebozytenproliferation und Sebogenese verantwortlich und stellen demzufolge den Motor für die Sebogenese dar.

Nach Choudhry, R. et al. (1992): Localisation of androgen receptors in human skin by immunohistochemistry: implications for the hormonal regulatin of hair growth, sebaceous gland and sweat glands, J. Endocr. 133, 467 - 475; Sawaya, M. E. (1992): Purification of androgen receptors in human sebocytes and hair, J. Invest. Dermat. 98, 92 - 96 ist Voraussetzung dafür unter anderem eine Interaktion mit Androgenrezeptoren, welche in der menschlichen Haut (Akne-Areale, wie Gesicht, obere Brustpartie, V-förmig am Rücken, Außenseiten der Oberarme), besonders den Talgdrüsen und Talgdrüsenfollikeln, lokalisiert sind.

Abhängig von der Androgensekretion und der peripheren Empfindlichkeit des Endorgans ist bei Aknepatienten die kontinuierliche Talgproduktion gesteigert und erreicht bei Acne conglobata ihre höchsten Werte.

In der Patentschrift DE-PS 43 21 957 C2 werden oral zu verabreichende Kombinationspräparate zur Aknetherapie beschrieben.

Nachteilig ist, daß durch die systemische Wirkung eine Stoffwechselbelastung bei Aknepatienten herbeigeführt wird.

Die Patentschrift DE-PS DE 36 15 396 A1 beschreibt Cyproteronacetat als Kombinationspräparat zur Behandlung der Kopfhaut und zur Förderung des Haarwachstums am Beispiel von Haarwassern.

Bei therapeutischem Einsatz von den Gestagenen Cyproteronacetat und Chlormadinonacetat beruht die Wirkung an den Talgdrüsen bzw. den Haarfollikeln auf einem zentral vermittelten antigonadotropen Effekt. Nachteilig dabei ist eine Aknetherapie bzw. eine Behandlung der Alopezie beim Mann mit diesen Wirkstoffen.

Nach Luderschmidt, Ch. (1995): Die Akne der Frau, gynäkol. prax., 19, 479 - 488 bewirken Cyproteronacetat und Chlormadinonacetat bei der Aknebehandlung der Frau eine periphere kompetitive Androgenrezeptorblockade.

In der Patentschrift EP 0 582 458 B1 wird eine pharmazeutische Formulierung von Aldacton, 3-(3-Oxo-7-$\alpha$-acetylthio-17$\beta$-hydroxy-androst-4-en-17$\alpha$-$\gamma$1), als Spironolacton zur topischen Behandlung von Akne vulgaris, Seborrhoe und Hirsutismus aufgezeigt.

Durch Verwendung eines Durchdringungsverstärkers und der Bereitstellung von therapeutisch nützlichen Konzentrationen am Wirkungsort, den Talgdrüsen, soll ein Überschuß des Arzneimittels im Blutkreislauf auf einem geringen Niveau gehalten werden, wodurch systemische Nebeneffekte vermieden werden sollen.

Auch hier besteht der Nachteil darin, daß aufgrund des zentral vermittelten antigonadotropen Effekts an den Talgdrüsen bzw. Haarfolikeln eine Aknetherapie bzw. eine Behandlung der Alopezie beim Mann mit diesen Wirkstoffen zu verneinen ist.

Ferner ist auch eine periphere kompetitive Androgenrezeptorblockade bei der Aknebehandlung der Frau schlußfolgernd.

Der Erfindung liegt die Aufgabe zugrunde, ein Mittel zur Behandlung der Haut mittels topischer Anwendung, besonders bei den Krankheitsbildern Akne oder Alopezie, aufzuzeigen, wobei die Permeation des Wirkstoffes durch die Haut verhindert werden soll, um unerwünschte systemische Nebeneffekte zu vermeiden.

Die Aufgabe wird erfindungsgemäß durch die Verwendung von Dienogest (17-Hydroxy-3-oxo-19-nor-17$\alpha$-pregna-4,9-dien-21-nitril entsprechend 17$\alpha$-Cyanomethyl-17$\beta$-hydroxy-13$\beta$-methyl-gona-4,9(10)-dien-3-on) ausschließlich oder in Kombination mit einem synthetischen oder natürlichen Estrogen oder Phytestrogen gelöst.

Vorteilhafte Ausgestaltungen der Erfindung sind in Anspruch 2 und 3 aufgezeigt.

Ferner wird die Aufgabe erfindungsgemäß auch durch die pharmazeutische Zusammensetzung gemäß Anspruch 4 gelöst.

Das natürliche Estrogen weist beispielsweise einen Bestandteil aus der Gruppe Estradiol, Estran, Estron, Estriol und anderen natürlichen Estrogenen oder mindestens eine Verbindung, die einen der vorgenannten Estrogenbestandteile nach Einnahme schnell abspaltet, auf.

Das synthetische Estrogen weist beispielsweise einen Bestandteil aus der Gruppe Ethinylestradiol, Mestranol und anderen synthetischen Estrogenen oder mindestens eine Verbindung, die einen der vorgenannten Estrogenbestandteile nach Einnahme schnell abspaltet, auf.

Der pharmazeutisch annehmbare Träger umfasst einen oder mehrere Puffer, Konservierungsmittel, Aromazusätze und Wirkstoffträger, um eine Creme, eine Salbe, eine Lotion, ein Liniment, ein Gesichtswasser, ein Haarwasser mit kosmetischem Aussehen zu erzielen.

Überraschenderweise konnte festgestellt werden, daß die vorliegenden pharmazeutischen Zubereitungen das Eindringen des Wirkstoffes oder der Wirkstoffkombination fördern, jedoch seinen/ihren Durchtritt durch die Haut und die Resorption einschränken.

Die pharmazeutischen Zubereitungen setzen den Wirkstoff bzw. die Wirkstoffkombination zügig und vollständig frei, fördern den Übertritt in das Gewebe, bei Vermeidung einer Absorption, und gehen dabei keine unerwünschten Wechselwirkungen (Interaktionen) mit dem biologischen Gewebe ein, wie sie beispielsweise für bestimmte Lösungsmittel und Tenside bekannt sind.

Es wurde festgestellt, daß Dienogest bei topischer Verabreichung eine signifikante Sebosuppression hervorruft, die eine Reduzierung der Androstandiolglucuronid-Konzentration und moderate kompetitive Hemmung der Androgenrezeptoren bewirkt.

Androstandiolglucuronid ist als Marker der peripheren Umwandlung von Testosteron in Dihydrotestesteron anzusehen. In vitro konnte an LNCaP-Zellen nachgewiesen werden, daß Dienogest auf dieser Ebene stärker antiandrogen als Cyproteronacetat (das zudem bei der Aknebehandlung mit einer peripheren kompetitiven Androgenrezeptorblockade behaftet sein kann) wirkt.

Bei ausgeprägter Akne ist die topische Anwendung einer Kombination von Dienogest und einem Estrogen in einer entsprechenden galenischen Form vorteilhaft, da bei einer Kombination Dienogest/ Estrogen, z. B. $17\alpha$-Estradiol oder Estriol, sich der suppressive Effekt potenziert.

Mit Beginn der topischen Behandlung kommt es insbesondere durch die direkte Wirkung der Estrogene auf die Talgproduktion zur Reduktion des Talgflusses. Gleichzeitig ist eine deutliche Reduktion der Talgdrüsenflächen nachweisbar. Androgenrezeptoren lassen sich in den geschrumpften Talgdrüsen nur noch selten oder gar nicht mehr nachweisen.

Nach drei bis vier Behandlungswochen bessert sich die Akne.

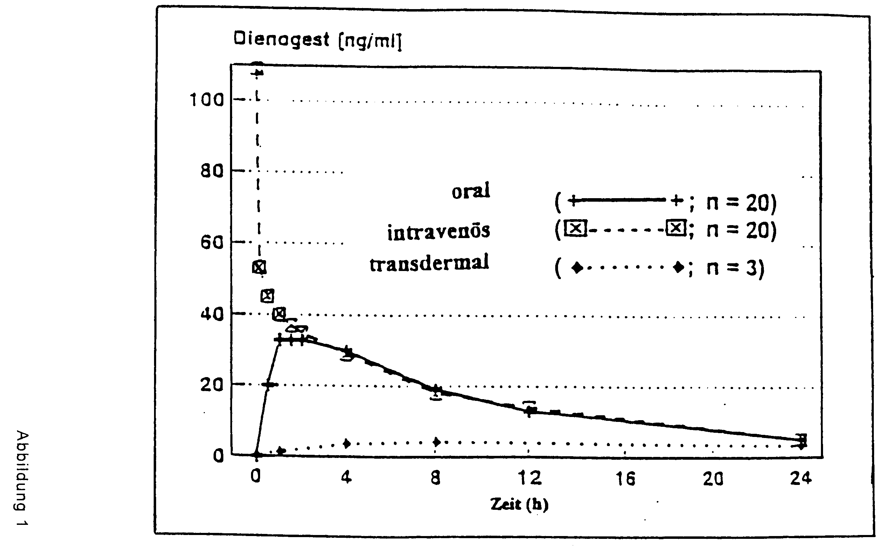

Abbildung 1 zeigt in Konzentrations-Zeit-Kurven die Serumkonzentration von 10 mg transdermal verabreichtem Dienogest im Vergleich zu 2mg oral bzw.intravenös verabreichtem Dienogest.

Nach transdermaler Verabreichung bewegen sich die nachgewiesenen Dienogestwerte im Serum im Bereich der Bestimmungsgrenze (1 ng/ml). Die durch die Überwindung der Hautbarriere und schlußfolgernd die Wirkung an den Talgdrüsen bzw. Haarfolikeln gegebenenfalls zu erwartenden ersten Anzeichen von systemischen Wirkungen von Dienogest wären erst bei Serumkonzentrationen ab 10 ng/ml gegeben.

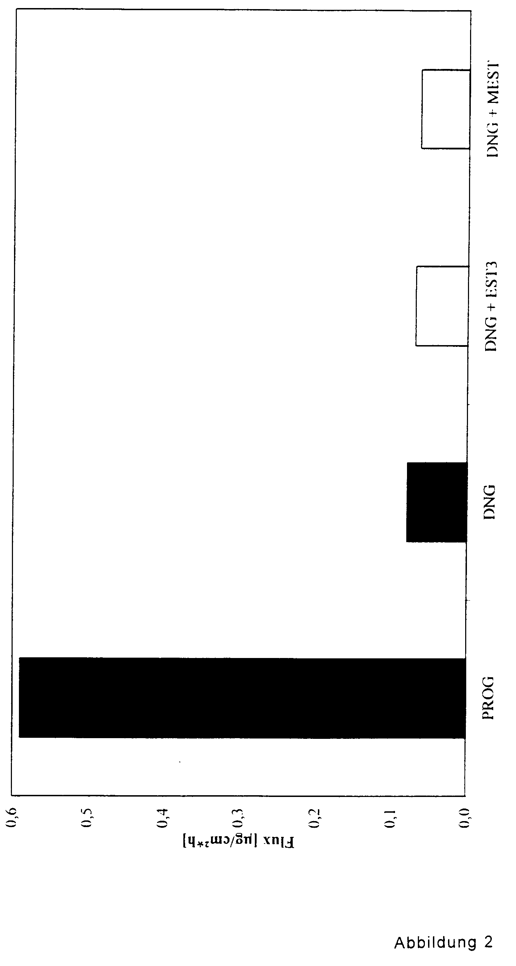

In Abbildung 2 ist der transdermale Flux (Permeationsrate $F_x$ bezogen auf eine gleiche Wirkstoffmenge) bei transdermaler Applikation von Dienogest, Dienogest in einer Zubereitung von Dienogest und Estriol und Dienogest in einer Zubereitung von Dienogest und Mestranol im Vergleich zum Progesteron aufgezeigt.

Es ist eine signifikante Reduzierung des transdermalen Flux, folglich eine Unterdrückung der Permeation und damit verbunden die Vermeidung von systemischen Nebenwirkungen von Dienogest, Dienogest in einer Zubereitung von Dienogest und Estriol und Dienogest in einer Zubereitung von Dienogest und Mestranol im Vergleich zum Progesteron ersichtlich.

Hervorzuheben ist, daß aufgrund der genannten Wirkungen eine transdermale Applikation der aufgezeigten Zubereitungen sowohl bei weiblichen und männlichen Patienten durch direkte Wirkung am Endorgan Haut möglich ist.

In der galenischen Zubereitung liegt der Wirkstoff in einer hydrophilen oder rückfettenden Grundlage in einem der Zubereitung angepaßten thermodynamischen Zustand vor.

Dabei wird der Wirkstoff so bereitgestellt, daß er in der rückfettenden oder hydrophilen Grundlage molekular dispers verteilt ist.

Bei Verwendung einer Zubereitung auf Basis einer rückfettenden Grundlage wird zusätzlich der Anregung zur Überproduktion von Fett entgegengewirkt, indem kontrolliert eine gewisse zur Rekonstituierung der Haut notwendige Menge Fett angeboten wird.

Im Falle einer hydrophilen Zubereitung (wäßrige Lösung, Hydrogel, Lotion oder O/W-Emulsion genügt es, das Gestagen in Ethanol oder einem ähnlichen Vehikel zu lösen und dieser Form zuzusetzen.

Bei einer rückfettenden Zubereitung (Lipogel oder eine zumindest teilweise Fette oder ähnliche Substanzen enthaltende Zubereitung, z. B. W/O-Emulsion) wird der Wirkstoff mit einer hydrophilen Komponente (H) kombiniert und mit ihr gemeinsam molekular dispers verteilt. Als hydrophile Komponente (H) kommen vorzugsweise Zucker oder zuckerähnliche Substanzen wie Saccharose, Lactose, Mannit, Zukkeralkohol, Harnstoff oder eine andere N-haltige Verbindung zur Anwendung.

Bei Kontakt mit Flüssigkeit der Haut (z. B. Schweiß) sorgt die hydrophile Komponente (H) dafür, daß der Wirkstoff sehr rasch in Lösung geht und in die Haut eindringt.

Weiterhin kann der Lösung des Wirkstoffes zusätzlich ein Stabilisator zugesetzt werden.

Der Stabilisator sorgt insbesondere im Falle der Lösung für eine ausreichende Beständigkeit des Wirkstoffes. Als Stabilisator kommen die grundsätzlich bekannten Antioxydantien, wie Tocopherol, aber auch Komplexbildner, Reduktionsmittel und/oder UV-Absorber in Betracht.

Ferner kann der Lösung des Wirkstoffes zusätzlich ein Permeationspromotor zugesetzt werden.

Der Permeationspromotor fördert zusätzlich das Eindringen des Wirkstoffes in die Haut. Als Permeationspromotor wird eine stickstoffhaltige Verbindung eingesetzt, beispielsweise ein Säureamid, ein Amin oder ein Alkaloid.

Für Dienogest ist Harnstoff als Permeationspromotor besonders geeignet.

Als Trägerstoffe/Hilfsstoffe für die lokal anwendbaren Zubereitungen mit Dienogest werden beispielsweise Öle (z. B. Erdnußöl und/oder Rizinusöl), Wachse (z. B. Bienenwachs, Hartwachs, flüssiges Wachs), Hydrogele, Ethanol, Wasser, Benzylbenzoat, Isopropylmyristat, Lezithin, Glycerol, Lanolin, Borsäure, Natriumtetraborat, Milchsäure, dickflüssiges Paraffin, Propylenglycol, entionisiertes Wasser oder Mischungen dieser Verbindungen miteinander verwendet. Mit Hilfe dieser Zubereitungen kann die kutane pharmakologische Wirkung des antiandrogen wirkenden Gestagen Dienogest und bei Kombination mit Estrogenen, die Reduzierung der Talgproduktion (Estrogeneffekt) in ausreichendem Maße in der Haut gewährleistet werden. Vorrangig kommt es dabei zu einer Sebosuppression, die zu einer Reduktion der Talgdrüsenflächen und zur Beseitigung von Komedonen führt. Eine nachteilige Beeinflussung der Wirksamkeit des Dienogest ist aufgrund der galenischen Zubereitungen nicht vorhanden. Mit einer Schädigung der behandelten Haut (Hornhaut, Haarfollikel) ist nicht zu rechnen.

Allergische und toxische Reaktionen sind durch den Wirkstoff, aber auch bei der Kombination mit Estrogenen sowie Zusatz- und Hilfsstoffen, nicht zu erwarten.

Zur Herstellung der pharmazeutischen Zubereitungen eignet sich ein geschlossenes System, in welchem der Wirkstoff vor Licht geschützt in einem ausgewogenen Verhältnis mit dem Permeationspromotor und dem Stabilisator kombiniert, höchstens kurz erwärmt und im gleichen Prozeß mit Hilfe eines Hochgeschwindigkeitsmischers in einem lipophilen oder hydrophilen Vehikel dispergiert werden kann.

Das Verfahren zur Herstellung der pharmazeutischen Zubereitungen wird im folgenden beschrieben.

Eine Mischung aus in der Dermatologie üblichen Hilfsstoffen (siehe Beispiele) wird vorgelegt. Die Hilfsstoffe werden in flüssiger oder fester Form eingetragen.

Bei einer hydrophilen Zubereitung wird der Wirkstoff oder die Wirkstoffkombination als Substanz oder gelöst in einem hydrophilen Vehikel zugeführt. Gemeinsam mit dem/der Wirkstoff/Wirkstoffkombination oder nachdem der/die Wirkstoff/Wirkstoffkombination vollständig in der Mischung aus Hilfsstoffen dispergiert worden ist, kann ein Stabilisator zugesetzt werden. Danach wird die gesamte Mischung homogenisiert. In Abhängigkeit von der Steifheit der Mischung erfolgt eine meist nur kurze Erwärmung. Meist wird jedoch gleich mit Beginn der Homogenisierung gekühlt.

Die Kühlrate ist wesentlich für die Stabilisierung des thermodynamischen Zustandes des/der Wirkstoffes/Wirkstoffkombination in der fertigen Zubereitung. Die Kühl- und die Rührgeschwindigkeit sind bei gegebener Rezeptur und Konzentration des Homogenisators/Stabilisators entscheidend für die Effektivität des/der Wirkstoffes/Wirkstoffkombination in der resultierenden Zubereitung.

Desweiteren hängt die Stabilität des/der Wirkstoffes / Wirkstoffkombination in der Zubereitung wesentlich von den bei der Herstellung herrschenden Temperaturen ab.

Es hat sich als vorteilhaft herausgestellt, wenn die Temperatur im Kessel und ebenso die Rotationsgeschwindigkeit der Rührwerke stufenlos regelbar sind und in einem definierten Verhältnis zur Heiz-/Kühlrate des den Kessel umgebenden Mantels stehen. Der Mantel kann durch Luft oder eine Flüssigkeit (Wasser) durchströmt werden.

Bei der Herstellung einer pharmazeutischen Zubereitung auf Basis einer rückfettenden Grundlage wird der/die pharmazeutische Wirkstoff/Wirkstoffkombination zunächst separat mit einer hydrophilen Komponente gemischt. Diese Mischung wird unter Ausschluß von Wasser, anderen Lösungsmitteln und/oder Tensiden zu mindest teilweisem Schmelzen erwärmt und anschließend unter schneller Abkühlung in ein Gas hinein dispergiert. Das so entstandene Zwischenprodukt wird nun der vorbereiteten Mischung aus in der Dermatologie üblichen Hilfsstoffen zugeführt und in der galenischen Zubereitung homogen verteilt. Entweder gemeinsam mit dem/der Wirkstoff/Wirkstoffkombination oder nachdem der/die Wirkstoff/Wirkstoffkombination vollständig dispergiert wurde, kann ein Stabilisator zugesetzt werden. Die Temperatur wird soweit erhöht, daß eine cremige Mischung gewährleistet ist. Nach erfolgtem Rühren wird die Temperatur der Zubereitung kontinuierlich abgesenkt. Die Kühlrate liegt dabei vorzugsweise bei etwa 1 °C/min.

Mit dem aufgezeigten Verfahren ist die Herstellung einer galenischen Zubereitung möglich, ohne daß der Wirkstoff mit aggressiven Lösungsmitteln oder Tensiden in Berührung kommt und ohne daß er längere Zeit dem ungehinderten Lichteinfall ausgesetzt ist. Die folgenden Beispiele veranschaulichen die Erfindung in nicht beschränkender Weise:

**Beispiel 1**

| Zusammensetzung der Lösung 1 (Gesichtswasser) | |
|---|---|
| Dienogest | 0,25 g |
| Rizinusöl | 0,4 g |
| Benzylbenzoat | 0,6 g |
| Ethanol (96 %) ad | 100,0 g |
| Farbstoffe, Parfumes | q.s. |

Herstellung:

Dienogest und Benzylbenzoat oder Isopropylmyristat werden in der angegebenen Menge Ethanol gelöst. Dabei kann die Mischung kurzzeitig auf 50 °C erwärmt werden. Danach muß die Mischung rasch auf 20 °C abgekühlt werden. Abschließend werden der Lösung die notwendigen Farb- und Aromastoffe in der angegebenen Menge Rizinusöl gelöst zugesetzt.

**Beispiel 2**

| Zusammensetzung Lösung 2 (Gesichtswasser) | |
|---|---|
| Dienogest | 0,2 g |
| Erdnußöl | 0,4 g |
| Lezithin | 2,5g |
| Isopropylmyristat | 2,0 g |
| Ethanol 96 % ad | 100,0 g |
| Farbstoffe, Parfumes | q.s. |

Herstellung:

Dienogest und Isopropylmyristat werden in der angegebenen Menge Ethanol gelöst. Dabei kann die Mischung kurzzeitig auf 50 °C erwärmt werden. Anschließend wird rasch auf 20 °C abgekühlt.
Zum Schluß werden der Lösung die erforderlichen Farb- und Aromastoffe, die zuvor mit Lezithin in dem Erdnußöl solubilisiert worden sind, als Mischung mit dem Öl und Lezithin zugesetzt.

**Beispiel 3**

| Zusammensetzung Hautcreme | | | |
|---|---|---|---|
| A | Bienenwachs | 4,5 g | |
| | Hartwachs | 9,5 g | |
| | Lanolin | 3,5 g | |
| | Isopropylmyristat | 8,0 g | |
| | flüssiges Wachs | 8,0 g | |
| | Glycerol | 4,0 g | 37,5 g |
| B | Dienogest | 2,2 g | |
| | Saccharose | 4,4 g | |
| C | entionisiertes Wasser | 55,6 g | |
| | Natriumtetraborat | 0,2 g | |
| | Borsäure | 1,5g | |
| | Milchsäure | 0,7 g | 62,2 g |
| D | Farbstoffe | | |
| | Konservierungsstoffe | | |
| | Parfumes | q.s. | |

Herstellung:

Die Stoffe des Ansatzes A werden auf 90 °C erwärmt, dabei geschmolzen und gemischt. In der Schmelze wird eine nach dem beschriebenen Herstellungsverfahren hergestellte feste Dispersion aus Dienogest und Saccharose 1 : 2 homogen verteilt. Dabei wird in dem erfindungsgemäßen Kessel unter folgenden Bedingungen gearbeitet:

| | |
|---|---|
| Kesseltemperatur bei Zugabe der festen Dispersion: | 40 °C |
| Umdrehungsgeschwindigkeit Abstreifer: | 1 U/min |
| Umdrehungsgeschwindigkeit langsamer Rührer: | 50 U/min |
| Umdrehungsgeschwindigkeit des Homogenisators: | 3450 U/min |
| Kühlrate: | 1 °C/min |

**Beispiel 4**

| Zusammensetzung Hautsalbe | | |
|---|---|---|
| A | Bienenwachs | 4,0 g |
| | Glycerolmonostearat | 9,5 g |
| | Lanolin | 4,0 g |
| | Isopropylmyristat | 8,0 g |
| | Paraffin (dickflüssig) | 8,0 g |
| | Propylenglycol | 4,0 g |
| B | entionisiertes Wasser | 20,0 g |
| | Natriumtetraborat | 0,2 g |
| C | Dienogest | 0,16 g |
| | Ethanol | 19,84 g |
| D | Farbstoffe | |
| | Konservierungsstoffe | |
| | Parfumes | q.s. |

Herstellung:

Die unter A aufgeführten Substanzen werden auf 90 °C erwärmt, geschmolzen und gemischt. Die Schmelze wird mit der ebenfalls auf 90 °C erwärmten Boraxlösung (Ansatz B) gemischt (Teilverseifung). Anschließend wird unter Rühren die Mischung aus A und B auf unter 50 °C abgekühlt, ehe die ethanolische Dienogestlösung (Ansatz C) zugemischt wird.

Zum Schluß werden der Mischung aus A, B und C die erforderlichen Farb-, Konservierungs- und Aromastoffe des Ansatzes D zugesetzt. Dabei wird unter Rühren auf 20 °C abgekühlt.

**Beispiel 5**

| Zusammensetzung Hautlotion | | |
|---|---|---|
| A | Dienogest | 0,5 kg |
| | Ethanol | 9,45 kg |
| | Glycerol | 0,05 kg |
| B | Sorbitanmonostearat | 1,0 kg |
| | Macrogolstearat | 1,5 kg |
| | Mittelkettige Triglyceride | 2,5 kg |
| | Kaliumsorbat | 0,05 kg |
| | Wasserfreie Citronensäure | 0,025 kg |
| | gereinigtes Wasser zu | 40,0 kg |

Herstellung:

In der Mischung B wird die Lösung A homogen verteilt. Dabei wird in dem bei der Beschreibung der allgemeinen Herstellungsweise erläuterten Kessel unter folgenden Bedingungen gearbeitet:

| Kesseltemperatur bei Zugabe der Lösung A: | 30 °C |
|---|---|
| Umdrehungsgeschwindigkeit Abstreifer: | 1 U/min |
| Umdrehungsgeschwindigkeit langsamer Rührer: | 90 U/min |
| Umdrehungsgeschwindigkeit des Homogenisators: | 3850 U/min |
| Kühlrate: | 1 °C/min |

**Beispiel 6**

| Zusammensetzung Lipogel | |
|---|---|
| Dienogest | 5,0 g |
| Wollwachsalkoholsalbe zu | 1000,0 g |

Herstellung:

Das mikronisierte Dienogest wird in der angegebenen Menge Wollwachsalkoholsalbe homogen verteilt.

**Beispiel 7**

| Lipogel + H mit Dienogest | |
|---|---|
| Dienogest | 5,0 g |
| Lactose | 45,0 g |
| Wollwachsalkoholsalbe zu | 1000,0 g |

Zunächst wird nach dem beschriebenen Herstellungsverfahren eine feste Dispersion aus Dienogest und Lactose 1 : 9 hergestellt. Diese feste Dispersion wird in der Wollwachsalkoholsalbe nach DAB 10 homogen verteilt. Dabei wird in einem analog kleineren Kessel, wie bei der Beschreibung der allgemeinen Herstellungsweise erläutert, unter folgenden Bedingungen gearbeitet:

| Kesseltemperatur bei Zugabe der festen Dispersion: | 40 °C |
|---|---|
| Umdrehungsgeschwindigkeit Abstreifer: | 2 U/min |
| Umdrehungsgeschwindigkeit langsamer Rührer: | 10 U/min |
| Umdrehungsgeschwindigkeit des Homogenisators: | 2050 U/min |
| Kühlrate: | 1 °C/min |

**Beispiel 8**

| Hydrogel mit Dienogest | |
|---|---|
| Dienogest | 5,0 g |
| Ethanol | 400,0 g |
| Methylhydroxyethylcellulose | 30,0 g |
| gereinigtes Wasser zu | 1000,0 g |

Herstellung:

Die angegebene Menge Dienogest wird in dem Ethanol gelöst und mit dem wäßrigen Methylhydroxyethylcellulose-Gel wie oben beschrieben homogen gemischt.

**Beispiel 9**

| Zusammensetzung Hydrogel mit Dienogest und 17$\alpha$-Estradiol | |
|---|---|
| Dienogest | 5,0 g |
| 17$\alpha$-Estradiol | 5,0 mg |
| Ethanol | 400,0 g |
| Methylhydroxyethylcellulose | 30,0 g |
| Gereinigtes Wasser zu | 1000,0 g |

Herstellung:

Die angegebenen Mengen Dienogest und 17$\alpha$-Estradiol werden in dem Ethanol gelöst und mit dem wäßrigen Methylhydroxyethylcellulose-Gel wie oben beschrieben homogen gemischt.

**Beispiel 10**

| Zusammensetzung Lipogel Dienogest plus Estriol | |
|---|---|
| Dienogest | 5,0 g |
| Estriol | 20,0 mg |
| Wollwachsalkoholsalbe zu | 1000,0 g |

Herstellung:

Das Dienogest und das Estriol werden in der angegebenen Menge in Wollwachsalkoholsalbe homogen verteilt.

**Beispiel 11**

| Zusammensetzung Lösung 1 (Haarwasser, Haarspray) | |
|---|---|
| Dienogest | 0,2 g |
| Rizinusöl | 0,4 g |
| Benzylbenzoat | 0,6 g |
| Ethanol 96 % ad | 100,0 g |
| Farbstoffe, Parfumes | q.s. |

Herstellung:

Dienogest und Benzylbenzoat oder Isopropylmyristat werden in der angegebenen Menge Ethanol gelöst. Dabei kann die Mischung kurzzeitig auf 50 °C erwärmt werden. Danach wird rasch auf 20 °C abgekühlt. Zum Schluß werden der Lösung die erforderlichen Farb- und Aromastoffe in der angegebenen Menge Rizinusöl gelöst zugesetzt.

**Beispiel 12**

| Zusammensetzung Dienogest-Haarbalsam mit haarfestigender Wirkung (Conditioner) | | |
|---|---|---|
| A | Cetyllactat | 2,0g |
| | Isopropylmyristat | 2,0 g |
| | Glycerolmonostearat Polyethylenglycol (PEG)- | 4,0 g |
| | 40-Stearat | 1,0g |
| | Cetyl-Stearylalkohol | 2,0 g |
| | Cetylalkohol | 1,0 g |
| B | Aloe vera als farbloses Gel | 30,0 g |
| | entionisiertes Wasser | 33,9 g |
| | Hydroxypropylmethylcellulose (HPMC) | 0,3 g |
| | Quat's | 3,4 g |
| | Mischung aus Quat-22 und Quat-26 (2 : 5) Milchsäure | 0,3 g |
| C | Dienogest | 0,23 g |
| | Ethanol | 19,75 g |
| D | Farbstoffe | |
| | Konservierungsstoffe | |
| | Parfumes | q.s. |

Herstellung:

Aus HPMC und Wasser wird ein Schleim hergestellt. Dem Schleim werden die übrigen Bestandteile des Ansatzes B zugesetzt. Die Mischung wird homogenisiert und danach auf 80 °C erwärmt (Wasserbad).

Ansatz B wird durch Mischen der Bestandteile bei 80 °C erhalten (Wasserbad). Dieser Ansatz wird mit dem gleichwarmen Ansatz A gemischt. Die Mischung A + B wird unter Rühren auf 50 °C abgekühlt. Der unter 50 °C abgekühlten Mischung A + B wird die ethanolische Lösung C unter Rühren zugesetzt. Dabei wird weiter abgekühlt. Zum Schluß werden der Mischung A + B + C die erforderlichen Farb-, Konservierungs- und Aromastoffe des Ansatzes D zugesetzt. Dabei wird unter Rühren auf 20 °C abgekühlt.

**Beispiel 13**

| Zusammensetzung Dienogest-Haarwasser | | |
|---|---|---|
| A | Cetyllactat | 1,0g |
| | Isopropylmyristat | 1,0 g |
| | Emulgator | 2,0 g |
| | Verdicker | 0,5 g |
| | Rückfetter | 0,5 g |
| B | Extr.Sabalae e fruct.spir.oleos | 30,0g |
| | Wasser | 30,0 g |
| | Verdicker | 0,5 g |
| | Emulgator | 3,0 g |
| | Milchsäure | 0,5 g |
| | Ethanol | 1,0 g |
| C | Dienogest | 0,3 g |
| | Ethanol | 19,7 g |
| D | Farbstoff | 0,2 g |
| | Konservierungsmittel | 0,7 g |
| | Parfumes | 0,1 g |
| | Ethanol | 9,0 g |

Herstellung:

Die Komponenten A bis D werden jeweils separat durch Mischen der angegebenen Bestandteile hergestellt. Dabei ist bei den Komponenten A und B geringfügig zu erwarmen. Anschließend werden die gegebenfalls vorher gekühlten Mischungen in einem geeigneten Gefäß vereinigt und das resultierende Haarwasser noch 30 min homogenisiert. Das Haarwasser wird vor der Abfüllung filtriert.

**Beispiel 14**

| Zusammensetzung Dienogest Haarcreme | | | | |
|---|---|---|---|---|
| A | Bienenwachs | 4,0 g | |
| | Hartwachs | 10,0 g | |
| | Lanolin | 3,5 g | |
| | Isopropylmyristat | 8,0 g | |
| | flüssiges Wachs | 8,0 g | |
| | Glycerol | 4,0 g | 37,5 g |
| B | Dienogest (mikronisiert) | 0,2 g | |
| C | entionisiertes Wasser | 60,0 g | |
| | Natriumtetraborat | 0,2 g | |
| | Borsäure | 1,5g | |
| | Milchsäure | 0,7 g | 62,2 g |
| D | Farbstoffe | | |
| | Konservierungsstoffe | | |
| | Parfumes | q.s. | |

Herstellung:

Die Bestandteile des Ansatzes A werden auf 90 °C erwärmt, dabei geschmolzen und gemischt. In der Schmelze wird das mikronisierte Dienogest homogen verteilt (Lichtschutz!). Dabei wird die Mischung unter Rühren auf mindestens 50 °C abgekühlt. Der unter 50 °C abgekühlten Mischung A + B wird die Wasserphase C unter Rühren zugesetzt. Dabei wird weiter abgekühlt.

Zum Schluß werden der Mischung A + B + C die erforderlichen Farb-, Konservierungs- und Aromastoffe des Ansatzes D zugesetzt. Dabei wird unter Rühren auf 20 °C abgekühlt.

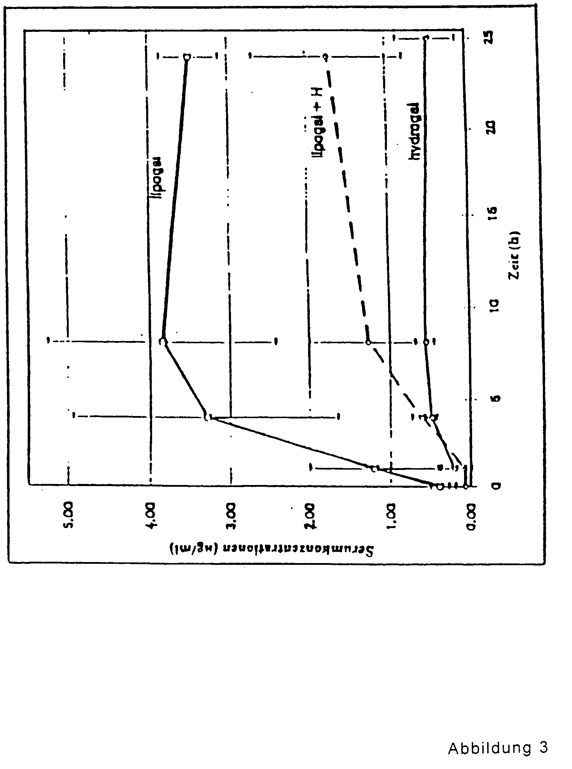

In Abbildung 3 ist die Serumkonzentration von Dienogest in Abhängigkeit von der Zeit am Beispiel eines Hydrogels (Hydrogel mit Dienogest), eines Lipogels + H (Lipogel mit Dienogest und hydrophilem Zusatz) und Lipogel mit Dienogest ohne hydrophilen Zusatz aufgezeigt.

Die pharmazeutischen Zubereitungen wurden jeweils 3 männlichen Probanden unter folgenden Bedingungen appliziert:

| Applizierte Masse: | 2 g (10 mg Dienogest) |
|---|---|
| Fläche: | Stirn |
| Einwirkzeit: | 3 h |

Danach wurden die Dienogest-Serumspiegel mittels RIA bestimmt.

Es ist ersichtlich, daß die ohnehin geringe Permeation des Wirkstoffes bei transdermaler Applikation eines Lipogels ohne hydrophilen Zusatz noch signifikant verringert werden kann durch hydrophilen Zusatz zur pharmazeutischen Zusammensetzung oder durch die Anwendung eines Hydrogels.

In einer Studie zur Penetration von Dienogest durch die intakte Haut konnte bei 9 männlichen Probanden festgestellt werden, daß der Wirkstoff (Formulierung Hydrogel) nicht in den Körper penetriert.

Auch bei 5 Aknepatienten konnte 12 und 24 Stunden nach zweimaliger Behandlung (10 mg Dienogest in 2 g Hautsalbe) kein Wirkstoff im Blutplasma nachgewiesen werden.

Im Rahmen von Wirksamkeitsstudien an 24 Frauen im Alter zwischen 18 und 45 Jahren und 18 Männern im Alter zwischen 24 und 46 Jahren wurde der Effekt einer 2,2 g dienogesthaltigen Hautcreme bei Aknepatienten und Patienten mit Seborrhoe geprüft.

Bei allen Patienten erfolgte die Verabreichung der Creme auf der rechten Stirnhälfte. Die linke Stirnhälfte diente als Kontrolle. Die Hautcreme wurde zweimal täglich (morgens und abends) auf das zu behandelnde Hautareal aufgetragen. Der Versuchszeitraum umfaßte 8 Wochen.

Bei allen Patienten konnte bereits nach 8 Behandlungstagen eine deutliche Reduzierung der Sebogenese nachgewiesen werden. Sowohl bei den Männern als auch bei den Frauen war nach 14- bis 21tägiger Behandlung eine ausgeprägte Reduzierung der Hautoberflächenlipide um 42 bzw. 47 % vorhanden.

Besonders war ein Rückgang des Anteils der Wachse zu beobachten. Diese Wachse sind von großer Bedeutung bei der Entstehung von Akne und Komedo.

Innerhalb der 4. bis 8. Behandlungswoche war eine deutliche Besserung bei vorhandener Akne nachweisbar. Bei 52 % der Aknepatienten waren nach 4 Behandlungswochen keine Symptome mehr vorhanden.

Nach 8 Therapiewochen zeigten nur noch 12 % der Patienten Erscheinungen von Akne. Dabei handelte es sich um stark ausgeprägte Krankheitsbilder zu Beginn der Therapie. Auch bei diesen Patienten war ein deutlicher positiver Krankheitsverlauf nachweisbar.

Dienogesthaltige pharmazeutische Zusammensetzungen zur transdermalen Applikation sind zur Behandlung der Haut, besonders bei Krankheitsbildern wie Acne vulgaris, Seborrhoe, androgenetisch bedingter Alopezie sowie Androgenisierungssymptomen der Frau und vasomotorischem Kopfschmerz einsetzbar.

Die Tests 1 - 5 zeigen beispielhaft die Anwendung erfindungsgemäßer Zusammensetzungen für die Behandlung der Akne und Seborrhoe:

**Test 1:**

Zur Behandlung der Seborrhoe wurde ein 0,25%iges dienogesthaltiges Gesichtswasser verwendet. Bei erhöhter Talgdrüsenproduktion im Gesichtsbereich empfiehlt sich eine zweimal tägliche Behandlung der betroffenen Hautareale. Das Mittel wurde über acht Wochen eingesetzt und zeigte eine ausgeprägte Sebosuppression.

**Test 2:**

Zur Aknetherapie wurde eine 3%ige dienogesthaltige Hautcreme auf die von Akne betroffenen Hautareale täglich zweimal aufgetragen. Die Creme wurde über 3 Monate verabreicht und zeigte bereits nach 4 Behandlungswochen eine sehr gute therapeutische Wirkung bei allen Schweregraden der Akne.

**Test 3:**

Zur Therapie der übermäßigen Talgproduktion in den Talgdrüsen der Haut wurde eine 0,2 % Dienogest enthaltenen Hautsalbe mindestens einmal pro Tag aufgetragen. Innerhalb von wenigen Minuten war die Salbe in die Haut eingezogen. Die Wirkung entsprach der unter Test 1 aufgezeigten Wirkung.

**Test 4:**

Als Präparat zur Therapie der Akne ist eine 0,5%ige dienogesthaltige Lotion verwendet worden. Die Lotion wurde täglich mindestens zweimal auf die Akneareale aufgetragen. Durch die schnelle Penetration in die Haut konnte eine sehr gute therapeutische Wirkung des Präparates, ebenso wie im Test 2 aufgezeigt, nachgewiesen werden.

**Test 5:**

Zur Behandlung der Akne ist ein 0,5%iges dienogesthaltiges Hydrogel, welches 5 mg 17$\alpha$-Estradiol in 1000 g Gel enthält, angewendet worden. Das Gel wurde über 3 Monate einmal pro Tag aufgetragen. Bereits nach 4 Behandlungswochen war eine deutliche Sebo-suppression mit teilweiser Beseitigung von Komedonen vorhanden.

Die Tests 6 - 8 zeigen beispielhaft die Anwendung erfindungsgemäßer pharmazeutischer Zusammensetzungen für die Behandlung des Haarausfalls und zur Förderung des Haarwuchses:

**Test 6:**

Bei Behandlung der gesamten Kopfhaut empfiehlt sich der Einsatz eines 0,2%igen Haarwassers/Haarsprays, wobei die Gesamtdosis von 20 ml pro Woche nicht überschritten werden sollte.

**Test 7:**

Bei postmenopausalem, androgenetisch bedingtem Haarausfall wird die Verwendung eines 0,5%igen Haarwassers/Sprays in einer Maximaldosierung bis 20 ml pro Woche vorgeschlagen.

**Test 8:**

Bei einem lokal begrenzten androgenetisch bedingten Haarausfall werden nur die betroffenen Kopfhautbezirke (Geheimratsecken` Stirnglatze, Hinterhauptglatze) mit Balsam bzw. Haarcreme oder Haarspray täglich oder mindestens 3 mal in der Woche behandelt.

Bei den Tests 6 - 8 konnte nach 3 Behandlungsmonaten ein therapeutischer Erfolg in Form der Zunahme des Haarwuchses nachgewiesen werden.

**Patentansprüche**

1. Verwendung von Dienogest ausschließlich oder in Kombination mit einem synthetischen oder natürlichen Estrogen oder Phytestrogen zur Herstellung eines Arzneimittels zur Behandlung der Haut mittels topischer Anwendung, wobei systemische Nebenwirkungen durch Verhinderung der Permeation des Wirkstoffes durch die Haut vermieden werden.

2. Verwendung nach Anspruch 1 zur Herstellung eines Arzneimittels, enthaltend 0,01 bis 4 Gew.-% an Dienogest.

3. Verwendung nach Anspruch 1 zur Herstellung eines Arzneimittels, enthaltend $5 \cdot 10^{-4}$ bis $2 \cdot 10^{-2}$ Gew.-% eines synthetischen oder natürlichen Estrogens.

4. Pharmazeutische Zusammensetzung zur Behandlung der Haut mittels topischer Anwendung von Dienogest in Kombination mit einem synthetischen oder natürlichen Estrogen oder Phytestrogen, wobei systemische Nebenwirkungen durch Verhinderung der Permeation des Wirkstoffes durch die Haut vermieden werden, umfassend

- 0,01 bis 4 Gew.-% Dienogest,
- $5 \cdot 10^{-4}$ bis $2 \cdot 10^{-2}$ Gew.-% synthetisches oder natürliches Estrogen,
- ein pharmazeutisch annehmbares Vehikel.

5. Pharmazeutische Zusammensetzung nach Anspruch 4, worin das Vehikel mindestens einen Puffer, mindestens ein Konservierungsmittel, mindestens einen Aromazusatz und/oder mindestens ein Excipiens enthält, das darauf eingestellt ist, eine Creme, eine Salbe, eine Lotion, ein Liniment, ein Gesichtswasser, ein Haarwasser mit kosmetischem Aussehen zu ergeben.

Dienagest [ng/ml]

oral ( +———————+ ; n = 20)

intravenös (⊠- - - - - -⊠; n = 20)

transdermal ( ♦·······♦ ; n = 3)

Zeit (h)

EP 0 765 663 A2

Abbildung 1

Abbildung 2

Abbildung 3